(19) Europäisches Patentamt
European Patent Office
Office européen des brevets

(11) Veröffentlichungsnummer: **0 071 738**
**B1**

(12) # EUROPÄISCHE PATENTSCHRIFT

(45) Veröffentlichungstag der Patentschrift: **19.02.86**

(21) Anmeldenummer: **82105665.2**

(22) Anmeldetag: **25.06.82**

(51) Int. Cl.⁴: **C 07 D 473/08** // A61K31/52

(54) **Neue Theophyllinderivate und Verfahren zu deren Herstellung.**

(30) Priorität: **20.07.81 CH 4739/81**

(43) Veröffentlichungstag der Anmeldung: **16.02.83 Patentblatt 83/7**

(45) Bekanntmachung des Hinweises auf die Patenterteilung: **19.02.86 Patentblatt 86/8**

(84) Benannte Vertragsstaaten: **AT BE DE FR GB IT LU NL SE**

(56) Entgegenhaltungen:
**EP - A - 0 021 103**
**EP - A - 0 023 350**
**DE - A - 1 915 979**
**FR - A - 1 456 392**

**EUROPEAN JOURNAL OF MEDICINAL CHEMISTRY - CHIMICA THERAPEUTICA, Band 14, Nr. 2, 1979, Seite 188, Paris (FR); E. DURANTI et al.: "7-Substituted theophyllines Part II. 3,4,5-Trimethoxybenzamidoderivatives".**
**IL FARMACO - EDIZIONE SCIENTIFICA, Band 34, Nr. 4, 1979, Seiten 284-91, Pavia (IT); E. DURANTI et al.: "7-Substituted theophyllines. Part I"**
**ARZNEIMITTELFORSCHUNG, Band 27, Nr. 1a, 1977,**

(73) Patentinhaber: **SIEGFRIED AKTIENGESELLSCHAFT, CH-4800 Zofingen (CH)**

(72) Erfinder: **Thiele, Kurt, Dr., Rebbergstrasse 47d, CH-4800 Zofingen (CH)**
Erfinder: **Geissmann, Felix, Brittnauerstrasse 28, CH-4800 Zofingen (CH)**
Erfinder: **Zirngibl, Ludwig, Dr., Eisengrubenweg 16, CH-4800 Zofingen (CH)**
Erfinder: **Jahn, Ulrich, Dr., Kirchmoosstrasse 13, CH-4800 Zofingen (CH)**

(74) Vertreter: **Jaeger, Klaus, Dr. et al, JAEGER & PARTNER Patentanwälte Bergstrasse 48 1/2, D-8035 München-Gauting (DE)**

(56) Entgegenhaltungen: (Fortsetzung)
**Seiten 4-14, Aulendorf (DE); K. KLINGLER: "Synthesen von bronchospasmolytisch wirksamen beta-Phenyläthyl-aminoalkyl-xanthinen".**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 071 738 B1**

## Beschreibung

Die Erfindung betrifft neue Theophillinderivate der Formel

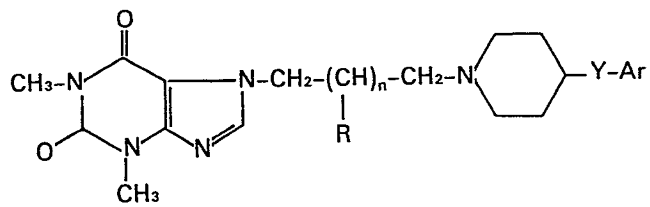

(I)

und ihrer Salze mit pharmazeutisch zulässigen Säuren, wobei

R Wasserstoff oder die Hydroxygruppe,

n die Zahlen null oder eins,

Y -O- oder

$-\underset{\underset{O}{\|}}{C}-$ und

Ar einen gegebenenfalls mit Niederalkyl oder Niederalkoxy mit 1 bis 3 C-Atomen, mit Halogen oder mit Halogenmethyl substituierten Phenyl, Furyl, Pyridyl, Thienyl, Pyrrolyl oder Imidazolyl bedeutet, sowie 7-{2-[4-(2-Furoyl)-1-piperazinyl]-ethyl}-theophyllin der Formel

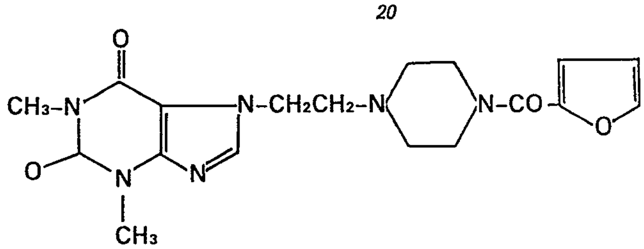

und

7-{3-[4-(2-Furoyl)-1-piperazinyl]-2-hydroxy-propyl}-theophyllin der Formel

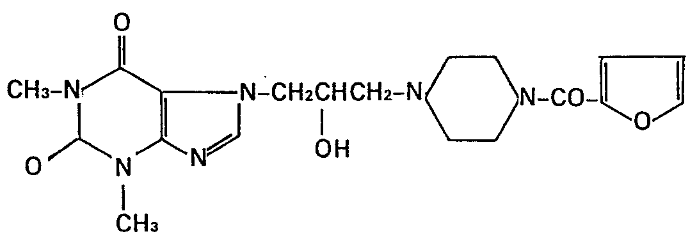

und deren Salze mit pharmazeutisch zulässigen Säuren.

Diese Verbindungen sind erhältlich nach dem ebenfalls Gegenstand der Erfindung bildenden Verfahren, welches dadurch gekennzeichnet ist, dass man in 7-Stellung mit einer Gruppe der Formel

$$-CH_2-\underset{\underset{R}{|}}{(CH)_n}-CH_2-Hal \qquad (II)$$

substituiertes Theophyllin (wobei R und n die oben genannte Bedeutung haben und «Hal» Chlor oder Brom bedeutet) mit einer Base der Formel

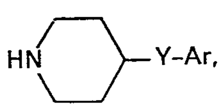

(IIIa)

bzw.

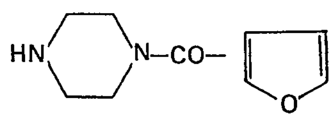

(IIIb)

für die beiden Piperazinderivate, zur Reaktion bringt.

Die Reaktion kann bei erhöhter Temperatur mit oder ohne Verwendung eines Lösungsmittels (wie z.B. Isopropanol) herbeigeführt werden. Beim Arbeiten ohne Lösungsmittel werden die Ausgangsstoffe so lange auf der Temperatur der Mischschmelze gehalten, bis periodisch entnommene Proben des Reaktionsgemisches bei Kontrolle mittels Dünnschicht-Chromatographie erkennen lassen, dass die Reaktion im wesentlichen abgeschlossen ist. Mit Vorteil benützt man dabei zur Bindung des abgespaltenen Halogenwasserstoffes einen Protonenakzeptor, wofür

man auch die als Ausgangsstoff benützte Base der Formel (III) in etwa 2- bis 3fachem Überschuss einsetzen kann.

Die Ausgangsstoffe der Formel (II) sind bekannt und grösstenteils im Handel erhältlich; gewünschtenfalls können sie auf bekanntem Wege [vgl. Arzneim.-Forsch. 27, 5 ff. (1977)] erhalten werden durch Kondensation von Theophyllin mit entsprechenden Alkylendihalogeniden bzw. Epichlorhydrin. Die als Ausgangsstoffe dienenden Basen der Formel (III) sind ebenfalls als Handelsprodukte in Form ihrer Hydrochlorid-Salze erhältlich, aus denen sie vor Verwendung zur erfindungsgemässen Reaktion durch Behandlung beispielsweise mit Alkalimetallhydroxid auf dem Fachmann bekannte Weise freizusetzen sind.

Es hat sich gezeigt, dass die erfindungsgemässen Theophyllinderivate überraschend vielseitige pharmakologische Wirkungen erkennen lassen.

Die pharmakodynamischen Eigenschaften der Xanthinderivate haben den Anlass gegeben, dass auch das Theophyllin und seine Abkömmlinge von der pharmazeutischen Chemie wegen ihrer Herz-/Kreislaufwirkung intensiv bearbeitet worden sind. Schon zu Beginn des Jahrhunderts hat man begonnen, das Theophyllin-Molekül, um dem Nachteil seiner geringen Wasserlöslichkeit zu begegnen, mit basischen Gruppen zu substituieren, und in der Folge ist eine grosse Zahl von Theophyllinderivaten synthetisiert worden, teils mit hydrophilen Substituenten in 7-Stellung, teils auch in Form von Salzen und Additions- bzw. Doppelverbindungen. Viele derselben haben als Arzneisubstanzen in die Therapie Eingang gefunden, wobei man von ihrer günstigen Beeinflussung des Kreislaufes unmittelbaren oder mittelbaren Gebrauch gemacht hat, um sie als Vaso-, Coronar-, Bronchodilatatoren oder Antiasthmatica einzusetzen, soweit es sich nicht um Salze bzw. Doppel-Verbindungen oder dgl. handelt, deren hinzugekommene Ionen bzw. Molekülkomponenten (wie etwa im Falle des Ephedrins spezifische eigene pharmakologische Wirkungen aufweisen und in diesem Sinne zusätzliche Wirkungskomponenten hinzutreten lassen oder die Wirkung des Theophyllins sogar überschatten und dominieren.

Vor kurzem sind nun in DE-OS 2 922 159 7-(4-Aminopiperidino-propyl)-theophylline mit antiallergischer und Antihistamin-Wirkung beschrieben worden. In ähnliche Richtungen weisen auch erfindungsgemäss Substanzen, für welche u.a. markante histamin-, serotonin- und bradykininantagonistische, blutdrucksenkende, antianaphylaktische und β-adrenerg stimulierende Eigenschaften nachgewiesen wurden und die sich durch dieses breite Wirkungsspektrum von den herkömmlichen Theophyllinderivaten deutlich abheben. Aufgrund der genannten pharmakodynamischen Eigenschaften lassen sie sich für die therapeutische Verwendung als Migränemittel, Broncholytica, Antiallergica, Antiphlogistica, Analgetica und Antihypertonica einsetzen. Als besonders vielversprechend erwiesen sich die Substanzen mit der Code-Bezeichnung Sgd 195-78 (vgl. nachfolgendes Beispiel 2) und Sgd 144-80 (vgl. Beispiel 3). Demgemäss haben nach dem derzeitigen Stand der Kenntnis diejenigen Verbindungen der Formel (I) als bevorzugt zu gelten, bei denen R ein Wasserstoffatom, n die Zahl null oder eins (vorzugsweise null), Y die Ketogruppe und Ar einen parasubstituierten Phenylrest, insbesondere die p-Fluorphenylgruppe, bedeuten.

Bei den Strukturformeln der nachfolgenden Beispiele bedeutet «Th» die Theophyllinylgruppe, die in 7-Position an die im jeweiligen Beispiel wiedergegebene Teilstruktur gebunden ist.

Weitere Theophyllinderivate der hier in Rede stehenden Art, insbesondere Piperazino-niederalkyltheophylline, sind auch aus den Druckschriften European Journal of Medicinal Chemistry-Chimica Therapeutica, Band 14, Nr. 2.1979, Seite 188, Il Farmaco-Edizione Scientifica, Band 34, Nr. 4, 1979, Seiten 284 bis 291, FR-A-1 456 392 und EP-A-23 350 bekannt. Diese Substanzen sind ebenfalls als Vaso- und Coronardilatatoren beschrieben, wobei sich in der zuletzt genannten Druckschrift auch ein Hinweis auf eine Antihistamin-Wirkung findet. Das breite Wirkungsspektrum der Theophyllinderivate gemäss der Erfindung zeigen jedoch auch diese bekannten Substanzen nicht.

Beispiel 1

7-{2-[4-(p-Tolyloxy)-piperidino]-ethyl}-theophyllin (Sgd 94-78)

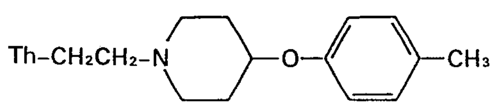

2,4 g (0,01 Mol) 7-(2-Chlorethyl)-theophyllin werden mit 3,8 g (0,02 Mol) 4-(p-Tolyloxy)-piperidin in festem Zustand sorgfältig gemischt und in einem Rundkolben auf dem Ölbad bei einer Badtemperatur von 90°C erhitzt. Dabei bildet sich bald eine klare Schmelze, worauf sich das Gemisch nach ungefähr 15 Minuten wieder verfestigt. Die Masse wird weiterhin auf gleicher Temperatur gehalten, bis die Kontrolle durch Dünnschicht-Chromatographie ($CHCl_3$ + 10% Methanol) erkennen lässt, dass die Reaktion beendet ist. Dies ist nach ungefähr 6 Stunden der Fall. Dann lässt man erkalten, versetzt mit Wasser und extrahiert mit Chloroform. Nach Trocknen des Extraktes über $MgSO_4$ wird das Chloroform abdestilliert. Der Rückstand wird zweimal aus Isopropanol umkristallisiert und ergibt dabei 3,0 g Produkt mit Smp. 121-122°C.

$C_{21}H_{27}N_5O_3$ 397,47

| | | | | |
|---|---|---|---|---|
| Ber.: | C 63,62; | H 6,61; | N 17,66; | O 12,11 |
| Gef.: | C 62,75; | H 6,96; | N 17,42; | O 13,02 |

Das als Ausgangsstoff benützte 7-(2-Chlorethyl)-theophyllin ist bekannt und durch Umsetzung von Theophyllin und Ethylendichlorid erhältlich. Das als freie Base eingesetzte 4-(p-Tolyloxy)-piperidin lässt sich aus dem entsprechenden Hydrochlorid durch Behandlung mit Natronlauge erhalten.

Beispiel 2
7-{2-[4-(p-Fluorbenzoyl)-piperidino]-ethyl}-theophyllin (Sgd 195-78)

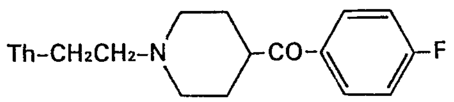

25,6 g (0,124 M) 4-(p-Fluorbenzoyl)-piperidin werden zusammen mit 15,0 g (0,062 M) 7-(2-Chlorethyl)-theophyllin in festem Zustand in einem Rundkolben während 1 Stunde auf 100°C erhitzt. Danach kühlt man auf 60°C und gibt unter Rühren Essigester zu, bis eine homogene Suspension vorliegt. Nach Abkühlen und Abfiltrieren des ausgeschiedenen Fluorbenzoylpiperidin-Hydrochlorids setzt man dem Filtrat zum Ausfällen des rohen Produktes 2n Salzsäure zu. Der durch Filtration abgetrennte Rückstand wird je zweimal mit Wasser und Essigester gewaschen, anschliessend mit Essigester überschichtet und mit 100 ml 2n Natronlauge versetzt. Nach Ausschütteln und Phasentrennung befindet sich die Hauptmenge des Produkts in der organischen Phase; zur Gewinnung weiterer Produktmengen wird die wässrige Phase zweimal mit je 100 ml Essigester ausgeschüttelt. Die vereinigten organischen Phasen werden mit gesättigter NaCl-Lösung gewaschen, mit Aktivkohle behandelt, über $MgSO_4$ getrocknet und filtriert. Aus dem Filtrat erhält man durch Abdestillieren des Lösungsmittels 20,4 g (80%) rohes kristallines Produkt. Zur weiteren Reinigung wird der Rückstand in 250 ml Tetrachlorkohlenstoff suspendiert und auf Rückflusstemperatur erhitzt. Die dabei entstehende dunkelbraune Lösung wird nach Behandlung mit Aktivkohle hellorange und liefert beim Abkühlen 14,5 g weisses Produkt mit Smp. 143–145°C, das sich im Dünnschicht-Chromatogramm als rein erweist.

$C_{21}H_{24}FN_5O_3$ 413,46

Ber.: C 61,00; H 5,85; N 16,94; F 4,60
Gef.: C 60,80; H 5,66; N 17,11; F 4,46

Durch fünfstündiges Erhitzen der im gleichen Verhältnis gemischten Ausgangsstoffe auf 120°C, anschliessendes Kochen in n-Propanol während 7 Stunden, entsprechende Aufarbeitung und Kristallisieren aus Tetrachlorkohlenstoff wurde ein gemäss Dünnschicht-Chromatogramm reines Produkt gleicher Zusammensetzung erhalten, welches bei 248°C unter Zersetzung schmilzt.

Ber.: C 61,00; H 5,85; N 16,94; F 4,60
Gef.: C 60,53; H 5,63; N 16,49; F 4,06

Beispiel 3
7-{3-[4-(p-Fluorbenzoyl)-piperidino]-propyl}-theophyllin (Sgd 144-80)

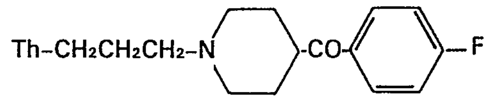

10,2 g 4-(p-Fluorbenzoyl)-piperidin und 16,5 g 7-(3-Chlorpropyl)-theophyllin werden in festem Zustand sorgfältig gemischt und in einem Rundkolben im Ölbad auf 100°C erhitzt. Das Gemisch ergibt dabei zunächst eine klare Schmelze, beginnt dann zu kristallisieren und verwandelt sich schliesslich in eine feste Masse. Nach 10 Minuten lässt man diese abkühlen, löst sie in Essigester auf und versetzt die Lösung mit 2n Salzsäure, wobei sich in der organischen Phase ein dunkles Öl abscheidet, das bei Stehenlassen über Nacht durchkristallisiert. Durch Umkristallisieren aus 100 ml Tetrachlorkohlenstoff erhält man als zunächst wiederum ölige, später auskristallisierende Substanz 7,4 g Produkt mit Smp. 114–119°C (Ausbeute: 43,5%).

$C_{22}H_{26}FN_5O_3$ 427,5

Ber.: C 61,81; H 6,13; N 16,38; F 4,45
Gef.: C 61,90; H 6,01; N 16,40; F 4,29

Das als Ausgangsprodukt benützte 7-(3-Chlorpropyl)-theophyllin lässt sich auf bekannte Weise beispielsweise durch Umsetzung von Theophyllin mit 1,3-Bromchlorpropan erhalten.

Beispiel 4
7-{3-[4-(p-Fluorbenzoyl)-piperidino]-2-hydroxy-propyl}-theophyllin-Hydrat (Sgd 145-80)

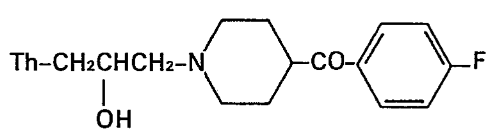

8 g 7-(3-Chlor-2-hydroxy-propyl)-theophyllin in Mischung mit 12,16 g 4-(p-Fluorbenzoyl)-piperidin werden im Ölbad während 1 Stunde auf einer Temperatur von 100°C gehalten, wobei die Viscosität der anfänglich dünnflüssigen, klaren Schmelze nach etwa 30 Min. zunehmend ansteigt. Nach Abkühlung wird das Gemisch unter intensivem Rühren während 30 Min. mit Essigester behandelt. Die entstandene Lösung wird durch Filtrieren vom ausgeschiedenen 4-(p-Fluorbenzoyl)-piperidin-Hydrochlorid abgetrennt und mit 2n Salzsäure versetzt. Nach Phasentrennung wird die wässrige Phase nach zweimaligem Ausschütteln mit Essigester durch Zugabe von konzentrierter Natronlauge alkalisch gestellt und zweimal mit Essigester extrahiert. Die aus dem Extrakt auskristallisierende Substanz zeigt nach Umkristallisieren aus Chloroform + Ethanol (9:1) und Trocknen im Hochvakuum einen Schmelzpunkt von 132–138°C.

$C_{22}H_{26}FN_5O_4 \cdot H_2O$ 461,5

Ber.: C 57,26; H 6,12; N 15,18; F 4,12
Gef.: C 57,54; H 6,15; N 15,52; F 4,10

Das als Ausgangsmaterial benützte 7-(3-Chlor-2-hydroxy-propyl)-theophyllin lässt sich auf bekannte Weise durch Reaktion von Theophyllin mit Epichlorhydrin erhalten.

Beispiel 5
7-{2-[4-(2-Furoyl)-1-piperazinyl]-ethyl}-theophyllin (Sgd 269-76)

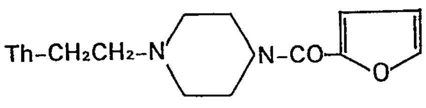

Ein Gemisch von 2 g (8,26 mMol) 7-(2-Chloraethyl)-theophyllin und 3,78 g (21 mMol) N-Furoylpiperazin wird auf dem Ölbad bei 100°C geschmolzen und 3 Stunden auf dieser Temperatur gehalten. Nach Abkühlen wird die erstarrte Masse in Methylenchlorid aufgelöst. Die Lösung wird mit Wasser extrahiert und anschliessend zweimal mit Salzsäure (10%) durchgeschüttelt. Die vereinigten wässrigen Phasen werden mit Natronlauge alkalisch gestellt und wiederum mit Methylenchlorid extrahiert, und der so erhaltene Extrakt wird über $MgSO_4$ getrocknet und eingeengt. Der Rückstand wird zweimal mit Wasser bei 60°C gewaschen und wieder in Methylenchlorid gelöst. Aus der Lösung erhält man nach Trocknung über $MgSO_4$, Abdampfen des Lösungsmittels und Umkristallisieren aus Essigester das reine Produkt mit Smp. 142–144°C.

$C_{18}H_{22}N_6O_4$ 386,41

Ber.: C 55,95; H 5,74; N 21,75
Gef.: C 55,86; H 5,84; N 21,71

Beispiel 6
7-{3-[4-(2-Furoyl)-1-piperazinyl]-2-hydroxy-propyl}-theophyllin (Sgd 123-77)

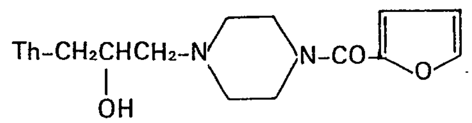

Eine Mischung von 4 g 7-(3-Chlor-2-hydroxy-propyl)-theophyllin mit 6,6 g N-(2-Furoyl)-piperazin wird 1 Stunde auf 120°C erhitzt; das Reaktionsgemisch wird in Chloroform gelöst und mit Wasser extrahiert. Die organische Phase wird über $MgSO_4$ getrocknet und eingeengt. Der Rückstand ergibt nach Umkristallisieren aus Isopropanol 2,8 g Produkt mit Smp. 161–163°C.

$C_{19}H_{24}N_6O_5$ 416,44

Ber.: C 54,80; H 5,81; N 20,18; O 19,12
Gef.: C 54,66; H 6,01; N 19,72; O 19,99.

**Patentansprüche für die Vertragsstaaten: BE, DE, FR, GB, IT, LU, NL, SE**

1. Theophyllinderivate der Formel

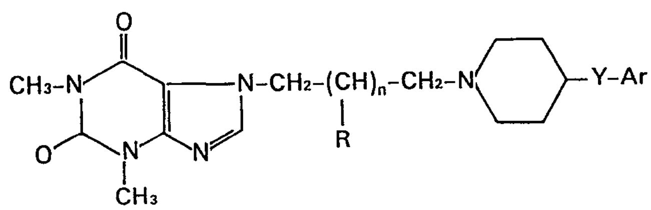 (I)

und ihrer Salze mit pharmazeutisch zulässigen Säuren, wobei
R Wasserstoff oder die Hydroxygruppe,
n die Zahlen null oder eins,
Y -O- oder

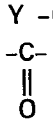 und

Ar ein gegebenenfalls mit Niederalkyl oder Niederalkoxy mit 1 bis 3 C-Atomen, mit Halogen oder mit Halogenmethyl substituiertes Phenyl, Furyl, Pyridyl, Thienyl, Pyrrolyl oder Imidazolyl bedeutet, sowie 7-{2-[4-(2-Furoyl)-1-piperazinyl]-ethyl}-theophyllin der Formel

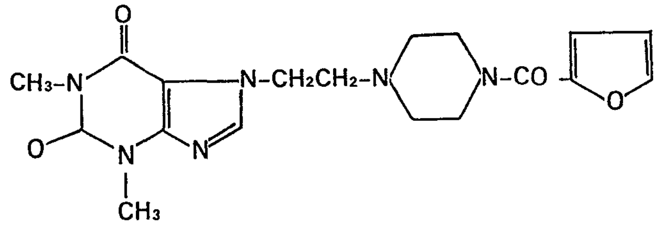

und
7-{3-[4-(2-Furoyl)-1-piperazinyl]-2-hydroxy-propyl}-theophyllin der Formel

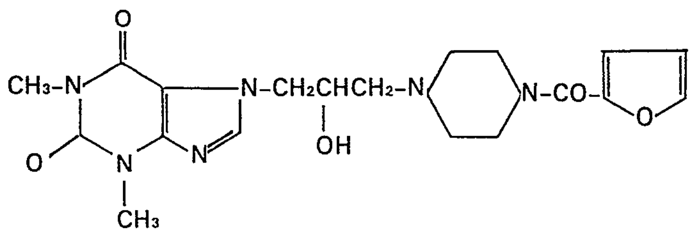

und deren Salze mit pharmazeutisch zulässigen Säuren.

2. Theophyllinderivate nach Anspruch 1, dadurch gekennzeichnet, dass R ein Wasserstoffatom, n null oder eins, Y die Ketogruppe und Ar die p-Fluorphenylgruppe bedeutet.

3. 7-{2-[4-(p-Fluorbenzoyl)-piperidino]-ethyl}-theophyllin.

4. Verfahren zur Herstellung der Theophyllinderivate von Anspruch 1, dadurch gekennzeichnet, dass man in 7-Stellung mit einer Gruppe der Formel

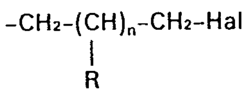

substituiertes Theophyllin (wobei R und n die oben genannte Bedeutung haben und «Hal» Chlor oder Brom bedeutet) mit einer Base der Formel

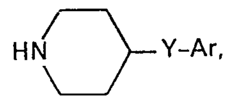

bzw.

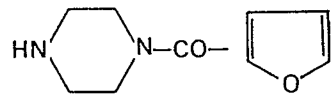

für die beiden Piperazinderivate, zur Reaktion bringt.

**Patentansprüche für den Vertragsstaat: AT**

1. Verfahren zur Herstellung von Theophyllinderivaten, dadurch gekennzeichnet, dass man zur Herstellung von Derivaten der Formel

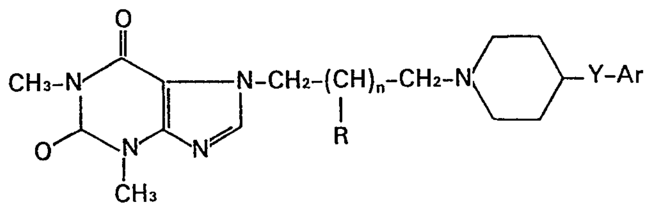
(I)

und ihrer Salze mit pharmazeutisch zulässigen Säuren, wobei

R Wasserstoff oder die Hydroxygruppe,

n die Zahlen null oder eins,

Y -O- oder

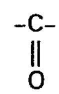 und

Ar ein gegebenenfalls mit Niederalkyl oder Niederalkoxy mit 1 bis 3 C-Atomen, mit Halogen oder mit Halogenmethyl substituiertes Phenyl, Furyl, Pyridyl, Thienyl, Pyrrolyl oder Imidazolyl bedeutet, sowie

7-{2-[4-(2-Furoyl)-1-piperazinyl]-ethyl}-theophyllin der Formel

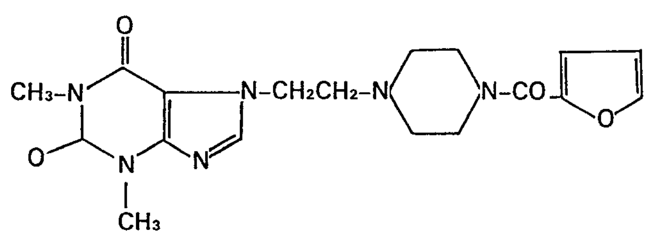

und 7-{3-[4-(2-Furoyl)-1-piperazinyl]-2-hydroxy-propyl}-theophyllin der Formel

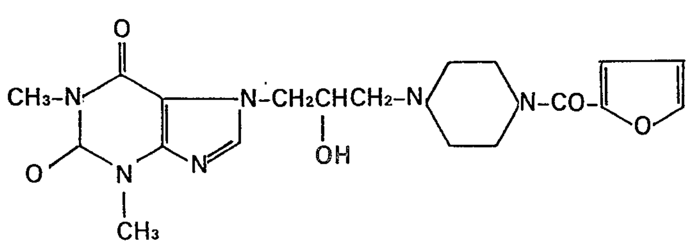

und deren Salze mit pharmazeutisch zulässigen Säuren, in 7-Stellung mit einer Gruppe der Formel

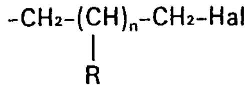

substituiertes Theophyllin (wobei R und n die oben genannte Bedeutung haben und «Hal» Chlor oder Brom bedeutet) mit einer Base der Formel

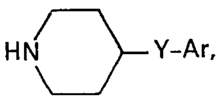

bzw.

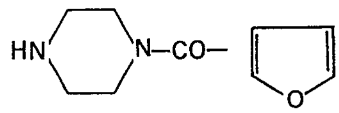

für die beiden Piperazinderivate, zur Reaktion bringt.

2. Verfahren nach Anspruch 1, dadurch gekennzeichnet, dass R ein Wasserstoffatom, n null oder eins, Y die Ketogruppe und Ar die p-Fluorphenylgruppe bedeutet.

3. Verfahren nach Anspruch 1, dadurch gekennzeichnet, dass das Theophyllinderivat 7-{2-[4-(p-Fluorbenzoyl)-piperidino]-ethyl}-theophyllin ist.

**Claims for the Contracting States BE, DE, FR, GB, IT, LU, NL, SE**

1. Theophylline derivatives having the formula

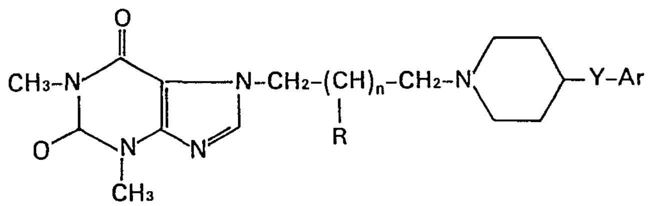
(I)

and their salts with pharmaceutically admissible acids wherein

R is hydrogen or a hydroxy group,

n is 0 or 1,

Y -O- or

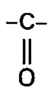 and

Ar is a radical such as phenyl, furyl, pyridyl, thienyl, pyrrolyl or imidazolyl eventually substituted by radicals such as lower alkyl group or a lower alkoxy group comprising 1 to 3 carbon atoms, halogen and halogen methyl as well as

7-{2-[4-(2-furoyl)-1-piperazinyl]-ethyl}-theophylline of formula

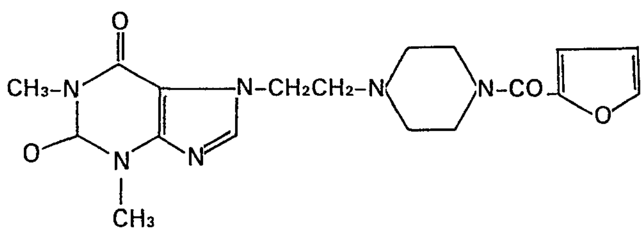

and

7-{3-[4-(2-furoyl)-1-piperazinyl]-2-hydroxy-propyl}-theophylline of formula

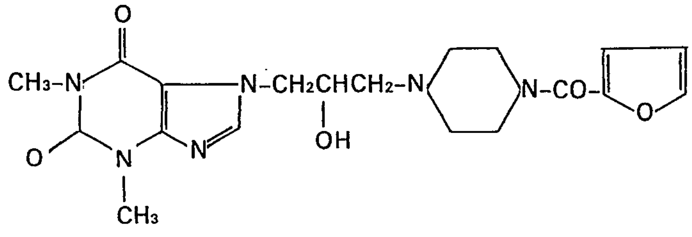

and their salts with pharmaceutically admissible acids.

2. Theophylline derivatives according to claim 1, wherein R is a hydrogen atom, n is 0 or 1, Y is a keto group and Ar is the p-fluorophenyl group.

3. 7-{2-[4-(p-fluoro-benzoyl)-piperidino]-ethyl}-theophylline.

4. A process for the preparation of theophylline derivatives according to claim 1, wherein a theophylline derivative substituted in position 7

by a radical of formula

$-CH_2-(CH)_n-CH_2-Hal$
|
R

(wherein R and n have the above mentioned meaning and Hal means chlorine or bromine) is brought in reaction with a base of formula

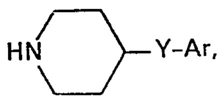

or

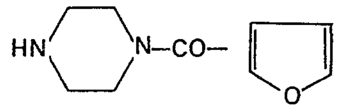

the base represented by the latter formula corresponding to said piperazine derivatives.

**Claims for the Contracting State AT**

1. A process for the preparation of theophylline derivatives, which comprises the preparation of derivatives of the formula

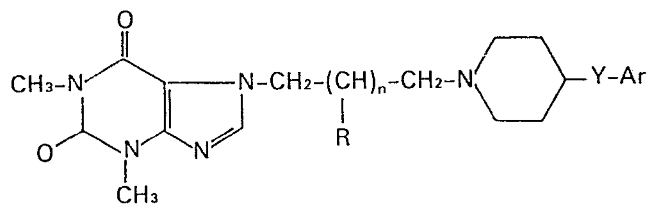

and their salts with pharmaceutically admissible acids, wherein
R is hydrogen or a hydroxyl group,
n is 0 or 1,
Y is -O- or

-C- and
|
O

Ar is a radical such as phenyl, furyl, pyridyl, thienyl, pyrrolyl or imidazolyl eventually substituted by radicals such as a lower alkyl group or lower alkoxy group comprising 1 to 3 carbon atoms, halogen or a halogen methyl as well as 7-{2-[4-(2-furoyl)-1-piperazinyl]-ethyl}-theophylline of the formula

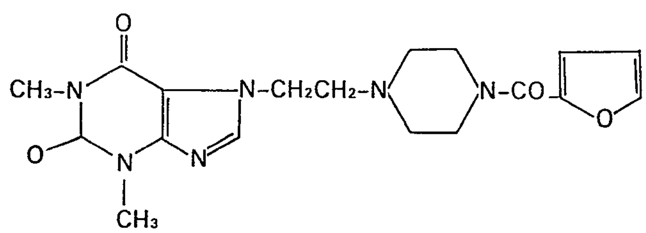

and
7-{3-[4-(2-furoyl)-1-piperazinyl]-2-hydroxy-propyl}-theophylline of the formula

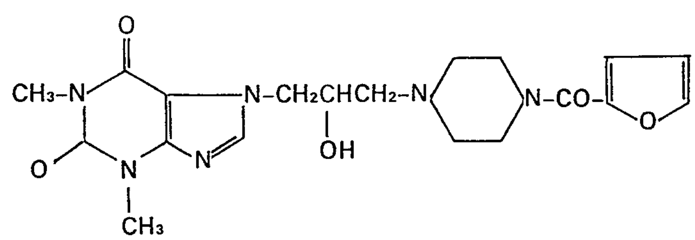

and their salts with pharmaceutically admissible acids, by reaction of a theophylline substituted in position 7 by a group of formula

$-CH_2-(CH)_n-CH_2-Hal$
|
R

(wherein R and n have the above mentioned meaning and Hal means chlorine or bromine), with a base of formula

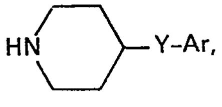

or

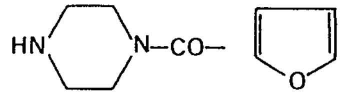

the base represented by the latter formula corresponding to said piperazine derivatives.

2. a process according to claim1, wherein R isa hydrogen atom, n is 0 or 1, Y is a keto group and Ar is the p-fluorphenyl group.

3. A process according to claim1, wherein theophylline derivative is 7-{2-[4-(p-fluorobenzoyl)-piperdino]-ethyl}[ théophylline

**Revendications pour les Etats contractants BE, DE, FR, GB, IT, LU, NL, SE**

1. Dérivés de la théophylline répondant à la formule:

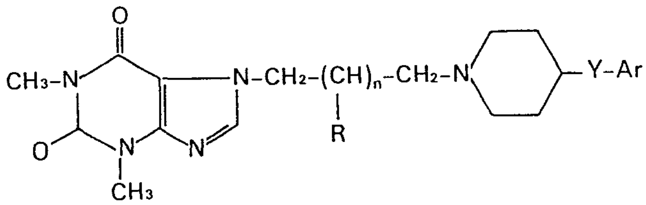

(I)

dans laquelle:
R représente l'hydrogène ou un radical hydroxy,
n désigne le nombre 0 ou le nombre 1,
Y représente -O- ou

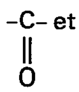 et

Ar représente un radical phényle, furyle, pyridyle, thiényle, pyrrolyle ou imidazolyle, éventuellement porteur d'un radical alkyle inférieur ou alcoxy inférieur contenant de 1 à 3 atomes de carbone, d'un halogène ou d'un radical halogénométhyle,
ainsi que les sels qu'ils forment avec des acides acceptables du point de vue pharmaceutique, et également la {[(furoyl-2)-4 pipérazinyl-1]-2 éthyl}-7 théophylline de formule:

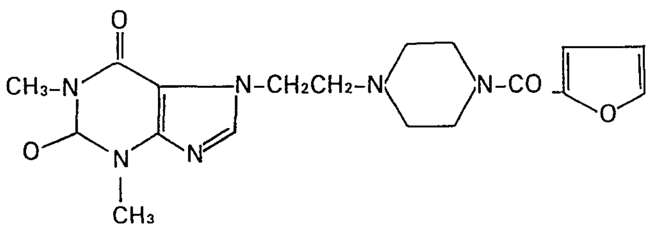

et la {[(furoyl-2)-4 pipérazinyl-1]-3 hydroxy-2 propyl}-7 théophylline de formule:

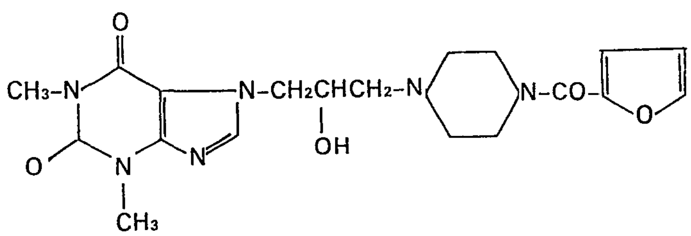

ainsi que les sels qu'elles forment avec des acides acceptables du point de vue pharmaceutique.

2. Dérivés de la théophylline selon la revendication 1, caractérisés en ce que R représente un atome d'hydrogène, n est égal à 0 ou à 1, Y représente un radical carbonyle et Ar représente un radical fluoro-4 phényle.

3. {[(Fluoro-4 benzoyl)-4 pipéridino]-2 éthyl}-7 théophylline.

4. Procédé de préparation de dérivés de la théophylline selon la revendication 1, caractérisé en ce qu'on fait réagir une théophylline portant, en position 7, un radical répondant à la formule:

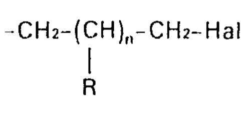

dans laquelle R et n ont les significations précédemment données et «Hal» représente le chlore ou le brome, avec une base de formule:

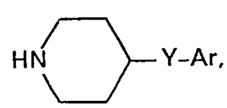

ou, lorsqu'on veut préparer l'un ou l'autre des deux dérivés de la pipérazine, avec une base de formule:

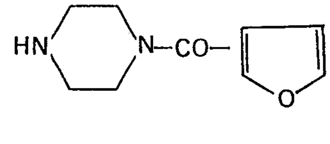

**Revendications pour l'Etat contractant: AT**

1. Procédé de préparation de dérivés de la théophylline, procédé caractérisé en ce que, pour préparer des composés répondant à la formule I:

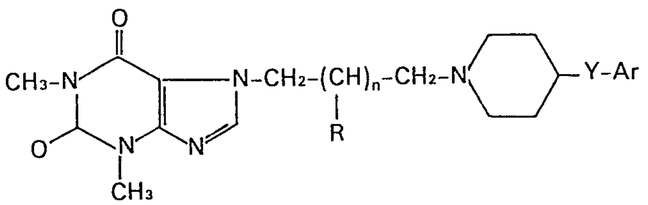

(I)

dans laquelle:
R représente l'hydrogène ou un radical hydroxy,
n représente le nombre 0 ou le nombre 1,
Y représente -O- ou

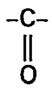 et

Ar représente un radical phényle, furyle, pyridyle, thiényle, pyrrolyle ou imidazolyle, éventuellement porteur d'un radical alkyle inférieur ou alcoxy inférieur contenant de 1 à 3 atomes de carbone, d'un halogène ou d'un radical halogénométhyle,
ainsi que les sels qu'ils forment avec des acides acceptables du point de vue pharmaceutique, et également la {[(furoyl-2)-4 pipérazinyl-1]-2 éthyl}-7 théophylline de formule:

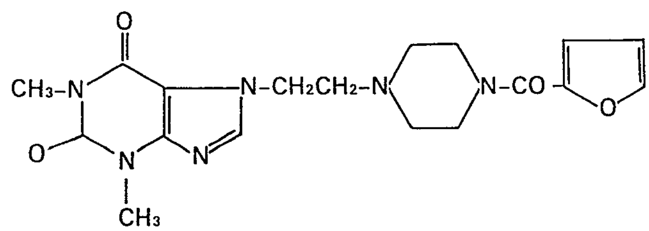

et la {[(furoyl-2)-4 pipérazinyl-1]-3 hydroxy-2 propyl}-7 théophylline de formule:

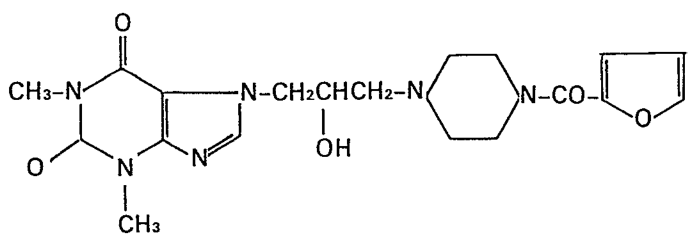

ainsi que les sels qu'elles forment avec des acides acceptables du point de vue pharmaceutique, on fait réagir une théophylline portant, en position 7, un radical répondant à la formule:

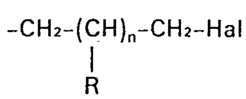

dans laquelle R et n ont les significations précédemment données et «Hal» représente le chlore ou le brome, avec une base de formule:

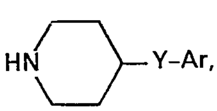

ou, lorsqu'on veut préparer l'un ou l'autre des deux dérivés de la pipérazine, avec une base de formule:

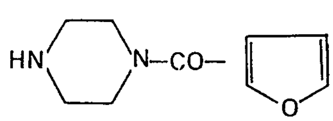

2. Procédé selon la revendication 1 caractérisé en ce que R représente un atome d'hydrogène, n est égal à 0 ou à 1, Y représente un radical carbonyle et Ar représente un radical fluoro-4 phényle.

3. Procédé selon la revendication 1 caractérisé en ce que le dérivé de la théophylline est la {[(fluoro-4 benzoyl)-4 pipéridino]-2 éthyl}-7 théophylline.